(19) Europäisches Patentamt
European Patent Office
Office européen des brevets

(11) Publication number: **0 049 671**
**A1**

(12) **EUROPEAN PATENT APPLICATION**

(21) Application number: 81401556.6

(22) Date of filing: 07.10.81

(51) Int. Cl.³: C 07 F 1/08
C 07 F 1/10, C 07 F 1/12
C 07 F 3/00, C 07 F 3/02
C 07 F 3/06, C 07 F 3/08
C 07 F 3/10, C 07 F 11/00
C 07 F 13/00, C 07 F 15/00
//C07C53/126, C07C53/124,
C07C53/122, C07C53/10,
C07C53/06

(30) Priority: 07.10.80 US 194849
17.12.80 US 217119
17.12.80 US 217120
08.12.80 US 214103

(43) Date of publication of application:
14.04.82 Bulletin 82/15

(84) Designated Contracting States:
AT BE CH DE FR GB IT LI LU NL SE

(71) Applicant: BioSystems Research, Inc.
109 W. Rainbow Drive
Salida Colorado 81201(US)

(72) Inventor: Lionelle, Joseph E.
Drawer 1037
Salida Colorado 81201(US)

(72) Inventor: Staffa, Jeffrey A.
Drawer 1037
Salida Colorado 81201(US)

(74) Representative: Lecca, Jean et al,
CABINET PLASSERAUD 84, rue d'Amsterdam
F-75009 Paris(FR)

(54) **New metal oxyalkylates, pharmaceutical compositions containing the same and process for preparing the same.**

(57) The invention is relative to new metal oxyalkylates, pharmaceutical compositions containing the same and a process for preparing the same.

The pharmaceutical compositions according to the invention contain as active substance a metal oxyalkylate of the general formula:

$$M_xO(R-CO_2)_6$$

wherein:

- M is a metal cation,
- x is an integer of 2 to 8,
- R is hydrogen or an alkyl group having preferably from 1 to 7 carbon atoms.

The compositions according to the invention are particularly useful in methods for combatting pathogenic micro-organisms such as bacteria, viruses or fungi.

EP 0 049 671 A1

# NEW METAL OXYALKYLATES, PHARMACEUTICAL COMPOSITIONS CONTAINING THE SAME AND PROCESS FOR PREPARING THE SAME

The invention relates to compounds active against pathogenic micro-organisms such as bacteria, viruses or fungi.

The present invention relates also to new compositions for use particularly in a method for combatting pathogenic micro-organisms such as bacteria, viruses or fungi and for treating viral, bacterial and/or fungal infections. These compositions are active in the treatment of Herpes Virus, including Herpes simplex virus, types I and II (herein after referred to as "HSV-1" and "HSV-2").

It also relates to a new process for making the compounds which are the active substance of said new compositions, and to the new compounds of the type of those obtained by said process.

The invention also relates to the active substances themselves, i. e. to the metal oxyalkylates, yet with the exception of the particular metal oxyalkylate consisting of zinc oxyacetate.

Zinc oxyacetate (zinc, hexakis (acetato) oxotetra) having the empirical formula $C_{12}H_{18}O_{13}Zn_4$ and the structural formula $Zn_4O(CH_3CO_2)_6$, has been described in the literature. See the Bulletin of the Chemical Society of Japan, March, 1954, volume 27, number 2, pages 112-114. In this literature article, and in others, the compound is prepared by slowly distilling powdered anhydrous zinc acetate in a high vacuum. Zinc oxyacetate sublimes gradually and is collected as a crystalline crust on a cool place in the container.

Preferred metal oxyalkylates according to the invention are those to which the following formula can be attributed :

$$M_xO(R-CO_2)_6$$

wherein :

- M is a metal cation,
- x is an integer of 2 to 8,
- R is hydrogen or alkyl group, preferably aliphatic, having preferably

from 1 to 7 carbon atoms, with the proviso that when $RCO_2$ is an acetate group, and x is 4, M is different from zinc cation.

Preferred new metal oxyalkylates according to the invention can be represented by the above mentioned formula wherein said metal cation is derived from the group consisting of zinc, beryllium, magnesium, chromium, manganese, iron, cobalt, nickel, palladium, platinum, copper, silver, gold, cadmium and mercury, with the proviso that when $RCO_2$ is an acetate group, and x is 4, M is different from zinc cation.

In the following description, the $RCO_2$ group will be designated by carboxylate group.

Preferred new metal oxyalkylates according to the invention can be represented by the above mentioned formula wherein $RCO_2$ is an aliphatic carboxylate group, preferably of 1 to 8 carbon atoms, preferably of the group including formiate, acetate, propionate and butyrate groups with the proviso that when $RCO_2$ is an acetate group, and x is 4, M is different from zinc cation.

Preferred new metal oxyalkylates according to the invention are manganese oxyalkylates, the structural formula of which has been confirmed to be :

$$MnO_4(R\text{-}CO_2)_6$$

and particularly manganese oxyacetate, the structural formula of which has been confirmed to be :

$$MnO_4(CH_3CO_2)_6$$

Further in accordance with the invention, the metal oxyalkylate provides an effective vehicle for supplying the metal to plants, animals and humans. The metal oxyalkylate is effectively applied to soil for assimilation by plants, and is effectively fed to animals or humans as a food supplement or in food compositions.

The invention also relates to therapeutic compositions which comprise, as active substance, an effective amount of at least a metal oxyalkylate, zinc oxyacetate being included among the contemplated active metal oxyalkylates.

According to the invention, the metal oxyalkylate can be represented by the following general formula :

$$M_xO(R-CO_2)_6$$

wherein :

- M is a metal cation,
- x is an integer of 2 to 8,
- R is hydrogen or an alkyl group, preferably aliphatic, having from 1 to 7 carbon atoms.

In a preferred embodiment of the invention, the metal cation M is derived from a metal selected from the group consisting of zinc, beryllium, magnesium, chromium, manganese, iron, cobalt, nickel, palladium, platinum, copper, silver, gold, cadmium and mercury.

Particularly, preferred metal cations are derived from zinc and manganese and their corresponding oxyalkyaltes have been found to have the structural formula :

$$M_4O(R-CO_2)_6$$

wherein M is derived from zinc or from manganese.

In another preferred embodiment of the invention, $RCO_2$ is a carboxylate group, preferably an aliphatic carboxylate group, preferably of 1 to 8 carbon atoms, preferably selected among formiate, acetate, propionate and butyrate groups.

The acetate group is particularly preferred.

According to an advantageous embodiment of the invention, in the active substance of the therapeutic composition according to the invention, M is derived from zinc or from manganese and $RCO_2$ preferably is an acetate group.

The therapeutic compositions of the invention preferably comprise an amount of metal oxyalkylate which is of 0.1 to 20 % by weight.

The active substance of the therapeutic compositions can generally be administered topically or systemically, preferably in a mixture with a pharmaceutical carrier.

For topical use, the active substance is preferably administered in an ointment or cream containing preferably from 0.1 to 20 % by weight of the active substance.

For systemic use, the active substance is preferably administered in a mixture with a pharmaceutically acceptable carrier comprising petrolatum or at least one alcohol having from 1 to 8 carbon atoms or a mixture thereof.

The carrier preferably comprises petrolatum and lauryl alcohol in a weight ratio of petrolatum/lauryl alcohol of from 1 : 10 to 10 : 1.

In preferred compositions of the invention, metal oxyalkylate is in an amount of 10 % by weight, petrolatum is in an amount of 80 % by weight, and lauryl alcohol is in an amount of 10 % by weight.

For systemic use, daily dosage is generally of about 15-50 mg of zinc for a person of average weight (70 kg).

It is also an object of the present invention to provide an efficient and economic new process for making the metal oxyalkylates.

As a matter of fact the process disclosed in the Bulletin of the Chemical Society of Japan, March, 1954, vol. 27, n° 2, p. 112-114, is a non-industrial process. This method has the drawbacks of consuming time and being expensive, that is why it is preferred to prepare metal oxyalkylates, including zinc oxyacetate according to the process of the invention.

The method according to the invention for making metal oxyalkylates comprises reacting a metal, a carboxylic acid, and hydrogen peroxide in an aqueous reaction mixture, precipitating the metal oxyalkylate, and recovering the precipitated metal oxyalkylate.

Preferred metals include beryllium, magnesium, chromium, manganese, iron, cobalt, nickel, palladium, platinum, copper, silver, gold, and zinc.

Preferred carboxylic acids include aliphatic carboxylic acids containing up to eight carbon atoms, such as formic, acetic, propionic and butyric acids.

The metal which is used as a reactant is free metal and is preferably in finely divided form, such as shot or powder, for facilitating reaction. The carboxylic acid is utilized in any convenient concentration, but relatively strong concentrations are preferred because it is preferred that the third reactant, hydrogen peroxide, is added in dilute aqueous solution and because it is preferred to minimize the total amount of water in the reaction mixture. Where the acid is acetic, glacial acid is readily available and readily usable. The amount of acid used is preferably not in excess of stoichiometric. Since it is a relatively simple matter to remove unreacted metal -which remains undissolved- it is preferred to use the metal in an amount in excess of stoichiometric relative to the acid. An excess of about 1.2 to 3 times stoichiometric is preferred.

Hydrogen peroxide is preferably used in excess of stoichiometric. A weight ratio of about 0.1 to 0.5 parts by weight of $H_2O_2$ per part by weight of metal is preferred. The hydrogen peroxide is preferably used in a dilute solution of up to 10 % $H_2O_2$. A 3 % solution is preferable in some situations such as when using glacial acetic acid, since the total amount of water in the aqueous reaction mixture is appropriate when introduced in this manner. The amount of water in the system can vary widely and is preferably at least sufficient to

prevent boiling without additional cooling. It is preferred that the amount of water is sufficient to prevent the temperature of the reaction mixture from rising about 90°C without additional cooling.

The reaction is preferably carried out at atmospheric pressure for reasons of economy and at temperatures below the boiling point of the reaction mixture. In general, a temperature of from room temperature up to about 130°C is preferred.

After the reaction is complete, excess metal is preferably removed by filtration or by decantation of the liquid phase. Precipitation is preferably achieved by adding acetone and cooling, but may be effected by careful evaporation of the aqueous reaction mixture. The amount of acetone is preferably about 1-5 volumes per volume of the aqueous phase and the aqueous phase is preferably cooled, or permitted to cool, to at least room temperature before the addition of the acetone in order to minimize evaporation of acetone. The solution is then preferably chilled, preferably to a temperature of less than 10°C (50°F), to precipitate the metal oxyalkylate. While chilling can be carried much lower, it is generally not necessary, in order to precipitate substantially all of the metal oxyalkylate, to cool below about - 1.1°C (30°F).

The precipitated metal oxyalkylate is recovered by filtration or the like and drying.

While the structure of the zinc oxyalkylate has been confirmed to be $Zn_4O(R\text{-}CO_2)$, and that of the manganese as $Mn_4O(R\text{-}CO_2)_6$, wherein R is hydrogen or alkyl, the structure of reaction products of all of the other metals and acids has not yet been confirmed. However, the following general information is attributed :

$$M_xO(R\text{-}CO_2)_6$$

where M is a metal, x is an integer of 2 to 8, and R is hydrogen or alkyl, preferably having from 1 to 7 carbon atoms.

Examples of the invention follow.

EXAMPLE 1 - Preparation of zinc oxyacetate

Into a 600 milliliters beaker are placed 50 g (0.765 gram-atoms) of zinc metal shot. Then 45 milliliters (0.767 moles) of glacial acetic acid is added. To this mixture is added 400 milliliters of 3 % hydrogen peroxide. This mixture is stirred at room temperature for one hour.

The reaction liquid mixture is decanted to remove the unreacted zinc shot. Then 1 200 milliliters of acetone is added to the decanted liquid, and the solution is cooled to 4.4°C (40°F). After one hour, the precipitate is filtered to yield 50 grams of white product (Hexakis (acetato) oxotetra zinc).

EXAMPLE 2 - Preparation of zinc oxyacetate

Into 2 000 milliliters beaker is placed 100 g (1.53 gram-atoms) of zinc metal shot. Then 90 milliliters (1.57 moles) of glacial acetic acid is added. To this mixture is added 800 milliliters of 3 % hydrogen peroxide. The mixture is heated to 94°C for 30 minutes with stirring. The unreacted zinc is removed, and the solution is chilled overnight in a larger container after adding 2 400 ml acetone to yield 115 g of white product (Hexakis (acetato) oxotetra zinc).

As mentioned above, the metal oxyalkylate can be added to soil as such or in admixture with a suitable carrier such as a fertilizer composition containing other fertilizer values such as N, P or K. The compound is also suitable for supplying metals to animals or humans and can be supplied in tablet or other convenient form in admixture with a suitable carrier such as a water soluble wax or other solid excipient. The compound can also be admixed with a food product for human or animal consumption, such as breakfast cereal, animal foods, and the like.

The amount of compound that is administered will, of course, depend on the amount of metal which is desired to be added to the plant or ingested by the animal or human. For agricultural use, the amount of metal which is

to be used may be determined by the amount by which the soil is considered deficient in a particular metal or by the amount by which the plant is considered deficient in that metal. Similarly, for animal or human use, the metal dosage may be determined by a dietary metal deficiency or by a metal deficiency in blood plasma, in body tissues, or the like. The dosage can vary widely for a particular metal, particularly for agricultural purposes. For zinc, dosages, in general, would typically be as follows :

| Use | Dosage |
|---|---|
| Agricultural........... | 2-12 ppm in the soil |
| Animal.................. | 50-250 mg per day |
| Human................... | 50-150 mg per day |

EXAMPLES 3-16

Example 1 is followed except that the zinc metal shot is replaced with 50 g of the following finely divided metals : 3 - beryllium ; 4 - magnesium ; 5 - chromium ; 6 - manganese ; 7 - iron ; 8 - cobalt ; 9 - nickel ; 10 - palladium ; 11 - platinum ; 12 - copper ; 13 - silver ; 14 - gold ; 15 - cadmium ; 16 - mercury. The recovered metal oxyacetate reaction products are useful, in the manner indicated above for the zinc oxyacetate reaction product, for supplying the metal to plants, animals and humans. The amount of the metal to be supplied will, of course, depend on the metal ; desirable amounts of each to be supplied are well known for particular applications.

EXAMPLES 17-20

Example 1 is followed except that the glacial acetic acid is replaced with an equivalent amount of the following acids : 17 - formic acid ; 18 - propionic acid ; 19 - n-butyric acid ; 20 - isobutyric acid. The recovered zinc oxyalkylate products are useful as indicated above.

EXAMPLE 21

The compound of Example 6 is analysed and found to have the following structural formula :

$$Mn_4O(CH_3-CO_2)_6.$$

The examples which follow illustrate the use of the compositions according to the invention as anti-virus, anti-bacteria, or anti-fungus agents.

EXAMPLE 22

A stock (1 %) solution is prepared by dissolving 0.1mg of zinc oxyacetate in 10 ml of water. A 1/2 dilution (0.5 % solution) is made by admixing 5 ml of the stock solution and 5 ml of water. A 1/4 dilution (0.25 % solution) is made by admixing 5 ml of the 0.5 % solution and 5 ml of water. A 1/10 dilution (0.1 % solution) is made by admixing 1 ml of the stock (1 %) solution and 9 ml of water. Fine filter paper discs of 1 cm diameter are placed in each of four clean petri dishes. In dish # 1, 0.1 ml of the 1 % solution is dropped onto each filter disc and allowed to air dry. The same is done in dish # 2 except that the 0.5 % solution is used. The same is done on dishes # 3 and # 4 except that the 0.25 and 0.1 % solutions, respectively, are used..One ml of a previously prepared 1 x $10^{-4}$ dilution of a broth bacterial culture (Staphylococcus aureus) is then spread evenly over the entire surface of four agar plates. At the 12 o'clock position on each of the agar plates, a disc from dish # 1 (1 % solution) is placed. Discs from dishes # 2, # 3 and # 4 are similarly placed at the 3 o'clock, 6 o'clock and 9 o'clock positions, respectively, of each of the four plates. Each disc is gently pressed to ensure good contact with the agar surfaces. For comparison purposes, a further stock solution of zinc sulphate (1 %) is made up and an identical series of tests made. Each plate is incubated overnight at 37°C and the area of inhibition -the clear area around each disc- is measured.

Results are as follows :

| Compound | Plate n° | Clear area (cm) | | | | Control |
|---|---|---|---|---|---|---|
| | | 1 % | 0.5 % | 0.25% | 0.01% | |
| Zinc oxyacetate | 1 | 1.85 | 1.70 | 1.32 | - | - |
| " | 2 | 1.80 | 1.52 | 1.35 | - | - |
| " | 3 | 1.78 | 1.61 | 1.40 | 1.25 | - |
| " | 4 | 1.85 | 1.72 | 1.42 | - | - |
| " | average | 1.82 | 1.64 | 1.37 | 0.31 | - |
| Zinc sulphate | 1 | 1.80 | 1.50 | 1.40 | - | N/A |
| " | 2 | 1.72 | 1.45 | 1.40 | 1.22 | N/A |
| " | 3 | 1.71 | 1.41 | 1.39 | - | N/A |
| " | 4 | 1.42 | 1.40 | 1.31 | 1.21 | N/A |
| | average | 1.66 | 1.44 | 1.37 | 0.61 | N/A |

The results show that zinc oxyacetate has bacteriostatic qualities comparable to those of zinc sulphate. At 1 % and 0.50 % concentration of zinc sulphate, a visible precipitate formed under each sensitivity disc.

EXAMPLE 23

Bread mold is innoculated on a nutrient plate. Discs of filter paper prepared as in Example 1 show good growth inhibition for the undiluted material and the 1 % solution. The less concentrated solution does not noticeably inhibit growth.

EXAMPLE 24

Evaluation of the compound is made using a simple neutralization of viral cytopathogenic effect (CPE) on Buffalo Green Monkey (BGM) cells using two tubes for each dilution of virus. The titer of Herpes simplex virus type 1 (HSV-1) is $10^{-4}$ based on $4^{+}$ (100 %) CPE, while that of HSV-2 is $10^{-2}$. In order to evaluate zinc oxyacetate, the compound (or the compound in equal weight admixture with lauryl alcohol) is incubated for 1 hour with the virus prior to making ten-fold serial dilutions and

innoculating the BGM tubes. The amount of virus used is 0.1 ml. The amount of test material used is 0.1 ml of a solution containing 0.1 % of Tween 80 and 0.1 % of the test material.

| Test | Titer based on 4 + (100 %) CPE | |
|---|---|---|
| | HSV-1 | HSV-2 |
| 1. Control | $10^{-4}$ | $10^{-2}$ |
| 2. Zinc oxyacetate | $10^{-3}$ (1 log inhibition) | 0 (2 log inhibition) |
| 3. 50 % zinc oxyacetate 50 % lauryl alcohol | $10^{-1}$ (3 log inhibition) | 0 (2 log inhibition) |

The data shows that the compound is effective in inhibiting the virus and that the compound in admixture with the lauryl alcohol carrier is substantially more effective than the compound *per se* in inhibiting the more virulent HSV-1 virus.

EXAMPLE 25

In a test similar to that of Example 23, zinc oxyacetate is tested for inhibition of four bread molds. The amount of zinc oxyacetate is 0.1 ml of a 1 % solution containing 0.1 % Tween 80. The amount of mold is 0.1 ml. Test results are indicated in the table.

| Mold | Size of cleared area (mm) |
|---|---|
| Candida albicans | 9 |
| Trichophyton mentagrophytes | 9 |
| Microsporum canis | 8 |
| Trichophyton rubrum | 5 |

The preferred carriers, for topical use, are petrolatum and alcohols of from 8-18 carbon atoms, preferably lauryl alcohol. An admixture of petrolatum and one or more of said alcohols may be used, preferably in a weight ratio of petrolatum to alcohol of 1 : 10 to 10 : 1. A suitable admixture is petrolatum 80 % and lauryl alcohol 20 %, by weight.

EXAMPLES 26-39

Results similar to those of Examples 22 to 25 are achieved by substituting the reaction products of acetic

acid, hydrogen peroxide, and one or more of the following metals according to the process of the invention :

5 - beryllium ; 6 - magnesium ; 7 - chromium ; 8 - manganese ; 9 - iron ; 10 - cobalt ; 11 - nickel ; 12 - palladium ; 13 - platinum ; 14 - copper ; 15 - silver ; 16 - gold ; 17 - cadmium ; 18 - mercury.

EXAMPLES 40-46

Manganese oxyacetate having the structural formula :

$$Mg_4O(CH_3CO_2)_6$$

is prepared according to the process of the invention. In this example, the manganese compound is compared, by plaque assays done on HSV-1 and HSV-2 with the zinc-lauryl acetate composition of Example 24. All of the tests, including those of Examples 47-52 which follow, are with test substances dissolved in 2 % FBS (fetal bovine serum)-MEM (Eagles Minimum Essential Medium). The solubility of the zinc containing compounds is poor and is reported as less than 1 mg/ml. The manganese compound is readily soluble and concentration is 1 mg/ml. Results are as follows :

A. HSV-2

| Example | HSV-2 | Manganese compound | Zn composition | Control |
|---|---|---|---|---|
| 40 | $10^{-1}$ | toxic | ND[1] | ND |
| 41 | $10^{-2}$ | 4 | 0 | 39 |
| 42 | $10^{-3}$ | 0 | 0 | 5 |

1 : not done

As indicated in the results, both substances inhibited HSV-2. However, the manganese compound was less effective than the zinc composition.

B. HSV-1

| Example | HSV-1 | Manganese compound | | | Zn composition | | | Control | | |
|---|---|---|---|---|---|---|---|---|---|---|
| | | 2 % (1) | 1.5 % | 0 % | 2 % | 1.5 % | 0 % | 2 % | 1.5 % | 0 % |
| 43 | $10^{-2}$ | Irr (2) | Irr | 90 | TNC (3) | TNC | TNC | ND (4) | ND | TNC |
| 44 | $10^{-3}$ | 46 | 45 | 11 | 24 | 105 | 31 | 115 | TNC | 34 |
| 45 | $10^{-4}$ | 5 | 5 | 4 | 16 | 6 | 5 | 10 | 38 | 6 |
| 46 | $10^{-5}$ | 0 | 0 | 0 | 0 | 0 | 0 | 3 | 3 | 5 |

(1) % methylcellulose in overlay medium (2 %, 1.5 % or 0 %)
(2) irregular results
(3) too numerous to count
(4) not done

The HSV-1 does not plaque as easily as the HSV-2 strain. HSV-1 has a tendancy to cause cell aggregate formation rather than distinct plaques. Consequently, two different concentrations of methylcellulose are used in the overlay medium, and finally, in the last trial a liquid overlay is used in an effort to get clear, distinct plaques. There are some variations in the results but, overall, with HSV-1, the Zn substance is less effective than the manganese compound in inhibiting HSV-1. This finding could be due to the lower solubility of the Zn material in 2 % FBS-MEM.

EXAMPLES 47-49

Cytopathogenic effect (CPE) trials are carried out on the manganese compound and zinc composition of Examples 40-46. As shown in the following results, these substances are not effective against the RNA viruses Echo 5 and Polio 1.

| Example | | Echo 5 | Polio 1 |
|---|---|---|---|
| 47 | Control | $10^{-6.25}$ (1) | $10^{-7}$ |
| 48 | Manganese compound | $10^{-6.0}$ | $10^{-7.5}$ |
| 49 | Zn composition | $10^{-6.5}$ | $10^{-7.5}$ |

(1) Approximate titer on BGM cells based on 4+ (100 %)CPE

EXAMPLES 50-51

CPE trials for the manganese compound of Examples 40-46 are conducted as in Examples 47-49 on HSV-1 and HSV-2. As is shown in the results which follow, the manganese compound is effective against HSV-2 but is probably not effective against HSV-1. The HSV-2 data are interesting because of the apparent viristatic action of the Mn compound rather than the viricidal activity seen with the Zn compound. Apparently at $10^{-1}$ dilution of HSV-2, enough Mn compound is present to inhibit the virus completely and cause an increase in the size of the BGM cells. At $10^{-2}$ and $10^{-3}$, one tube in each dilution showed early inhibition of virus but later the virus manifested itself in the typical syncytial formation.

| | HSV-1 | HSV-2 |
|---|---|---|
| Control | $10^{-3.5}$ (1) | $10^{-2.5}$ |
| Mn compound | $10^{-3.0}$ | Inhibition |

(1) approximate titer on BGM cells based on 4+ (100 %) CPE

EXAMPLE 52

CPE trials for the manganese compound and zinc composition of Examples 40-46 are conducted as in

Examples 47-51 on a DNA virus, Adenovirus type 4, using KB cells (human continuous cell line). The data clearly indicates that neither the test compound nor Zn carrier are effective in inhibiting the CPE of Adenovirus type 4 on KB cells. The titer in all cases is $10^{-3}$.

CLAIMS (EXCEPT AT)

1. A composition for use particularly in a method for combatting pathogenic micro-organisms, such as bacteria, viruses or fungi, comprising as active substance an effective amount of a metal oxyalkylate.

2. A composition according to claim 1, wherein said metal oxyalkylate has the general formula :

$$M_xO(R-CO_2)_6$$

wherein :

- M is a metal cation,
- x is an integer of 2 to 8,
- R is hydrogen or an alkyl group having preferably from 1 to 7 carbon atoms.

3. A therapeutic composition according to anyone of claims 1 and 2, wherein said metal cation M is derived from a metal selected from the group consisting of zinc, beryllium, magnesium, chromium, manganese, iron, cobalt, nickel, palladium, platinum, copper, silver, gold, cadmium and mercury.

4. A therapeutic composition according to anyone of claims 1 to 3, wherein $RCO_2$ is a carboxylate group selected from the formiate, acetate, propionate and butyrate groups.

5. A therapeutic composition according to claim 2, wherein M is derived from zinc or from manganese, x is 4 and $RCO_2$ is an acetate group.

6. A therapeutic composition according to anyone of claims 1 to 5, wherein the metal oxyalkylate is present in an amount of 0.1 to 20 % by weight.

7. A therapeutic composition according to anyone of claims 1 to 6, which is administered in a mixture with a pharmaceutically acceptable carrier comprising petrolatum or at least one alcohol having from 1 to 8 carbon atoms or a mixture thereof.

8. A therapeutic composition according to anyone of claims 1 to 7, wherein said carrier comprises petrolatum and lauryl alcohol in a weight ratio of petrolatum/lauryl alcohol of from 1 : 10 to 10 : 1.

9. A therapeutic composition according to anyone of claims 1 to 8, wherein metal oxyalkylate is in an amount of 10 % by weight, petrolatum is in an amount of 80 % by weight, and lauryl alcohol is in an amount of 10 % by weight.

10. A therapeutic composition according to anyone of claims 1 to 9, in a form and a dosage suitable for topical administration.

11. A therapeutic composition according to claim 1, in a form and a dosage suitable for systemical, notably, oral administration.

12. A method of preparing a metal oxyalkylate which comprises reacting the corresponding metal, a carboxylic acid, preferably having from 1 to 7 carbon atoms and hydrogen peroxide in an aqueous reaction mixture and recovering the metal oxyalkylate.

13. A method according to claim 12 wherein said metal is selected from the group consisting of zinc, beryllium, magnesium, chromium, manganese, iron, cobalt, nickel, palladium, platinum, copper, silver, gold, cadmium and mercury.

14. A method according to anyone of claims 12 and 13, wherein said acid is selected from the group consisting of formic, acetic, propionic and butyric acid.

15. A method according to anyone of claims 12 to 14, wherein the amount of hydrogen peroxide is from 0.1 to 0.5 parts by weight of metal and that preferably said hydrogen peroxide is added to the reaction mixture as a dilute aqueous solution.

16. A method according to anyone of claims 12 to 15, wherein the temperature of said reaction mixture is maintained below about 130°C.

17. A metal oxyalkylate with the proviso that when the carboxylate group is acetate, the metal is not zinc.

18. A metal oxyalkylate according to claim 17 having the formula :

$$M_xO(R-CO_2)_6$$

wherein :

- M is a metal cation,
- x is an integer of 2 to 8,
- R is hydrogen or an alkyl group having from 1 to 7 carbon atoms.

19. A metal oxyalkylate according to claim 18, wherein said metal cation is derived from the group consisting of zinc, beryllium, magnesium, chromium, manganese, iron, cobalt, nickel, palladium, platinum, copper, silver, gold, cadmium and mercury.

20. A metal oxyalkylate according to anyone of claims 18 and 19, wherein the $RCO_2$ group consists of formiate, acetate, propionate and butyrate.

21. A metal oxyalkylate according to anyone of claims 18 to 20, wherein M is manganese, $RCO_2$ is an acetate group and x is 4.

CLAIMS FOR AT

1. A method of preparing a metal oxyalkylate which comprises reacting the corresponding metal, a carboxylic acid, preferably having from 1 to 7 carbon atoms and hydrogen peroxide in an aqueous reaction mixture and recovering the metal oxyalkylate.

2. A method according to claim 1 wherein said metal oxyalkylate has the general formula :

$$M_xO(R-CO_2)_6$$

wherein :

- M is a metal cation,
- x is an integer of 2 to 8,
- R is hydrogen or an alkyl group having preferably from 1 to 7 carbon atoms.

3. A method according to anyone of claims 1 and 2, wherein said metal is selected from the group consisting of zinc, beryllium, magnesium, chromium, manganese, iron, cobalt, nickel, palladium, platinum, copper, silver, gold, cadmium and mercury.

4. A method according to anyone of claims 1 to 3, wherein said acid is selected from the group consisting of formic, acetic, propionic and butyric acid.

5. A method according to anyone of claims 1 to 4, wherein the amount of hydrogen peroxide is from 0.1 to 0.5 parts by weight of metal and that preferably said hydrogen peroxide is added to the reaction mixture as a dilute aqueous solution.

6. A method according to anyone of claims 1 to 5, wherein the temperature of said reaction mixture is maintained below about 130°C.

0049671

European Patent Office

# EUROPEAN SEARCH REPORT

Application number
EP 81 40 1556

| Category | Citation of document with indication, where appropriate, of relevant passages | Relevant to claim | CLASSIFICATION OF THE APPLICATION (Int. Cl.³) |
|---|---|---|---|
| X | FR - A - 1 483 233 (THE BRITISH PETROLEUM)<br>* Page 1, column 2, paragraph 2; page 2, table 1 *<br>-- | 17-19 | C 07 F 1/08<br>1/10<br>1/12<br>3/00<br>3/02<br>3/06<br>3/08<br>3/10<br>11/00<br>13/00<br>15/00<br>15/02<br>15/04<br>./. |
| X | JOURNAL OF INORGANIC NUCLEAR CHEMISTRY, vol. 39, 1977<br>T.J. CARDWELL et al. "Gas chromatography of some beryllium oxycarboxylates", pages 2139-2141.<br>* Whole document *<br>-- | 17-20 | TECHNICAL FIELDS SEARCHED (Int. Cl.³) |
| X | CHEMICAL ABSTRACTS, vol. 56, no. 9, April 30, 1962<br>abstract 9679c<br>Columbus, Ohio, US<br>K.N. SEMENENKO et al. "Heats of sublimation of some beryllium oxy-salts"<br>& ZHUR. NEORG. KHIM. 6, 2025-28 (1961)<br>* Abstract *<br>-- | 17-20 | C 07 F 1/08<br>1/10<br>1/12<br>3/00<br>3/02<br>3/06<br>3/08<br>3/10<br>11/00<br>13/00<br>15/00<br>15/02<br>15/04<br>15/06<br>./. |
| X | CHEMICAL ABSTRACTS, vol. 57, no. 8, October 15, 1962,<br>abstract 9434c<br>Columbus, Ohio, US<br>K.N. SEMENENKO et al. "Oxysalts of beryllium of alicyclic and aliphatic acids"<br>& ZH NEORGAN. KHIM. 7, 1512-15 (1962)<br>* Abstract *<br>--<br>./. | 17-20 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons<br>&: member of the same patent family, corresponding document |

The present search report has been drawn up for all claims

| Place of search | Date of completion of the search | Examiner |
|---|---|---|
| The Hague | 15-12-1981 | LI VOTI |

European Patent Office

# EUROPEAN SEARCH REPORT

Application number
EP 81 40 1556
- 2 -

## DOCUMENTS CONSIDERED TO BE RELEVANT

| Category | Citation of document with indication, where appropriate, of relevant passages | Relevant to claim |
|---|---|---|
| X | NATURE, vol. 163, no. 4136, February 5, 1949<br>T. WATANABE et al. "Polymorphism of beryllium oxyacetate"<br>pages 225-226<br>* Whole article *<br>-- | 17-20 |
| X | RECUEIL DES TRAVAUX CHIMIQUES DES PAYS BAS, vol. 88, no. 5, May 1969<br>L.W. HESSEL et al. "The crystal structure of "anhydrous manganic acetate"" pages 545-552.<br>* Whole article *<br>-- | 17-20 |
| X | CHEMICAL ABSTRACTS, vol. 83, no. 8, August 25, 1975<br>page 791, abstract 70093v<br>Columbus, Ohio, US<br>J. CHARALAMBOUS et al. "Mass spectra of tetracobalt (II) and tetrazinc (II) $\mu$ 4-oxo-hexa-$\mu$-carboxylates (basic cobalt and zinc carboxylates)"<br>& INORG. CHIM. ACTA, 1975, 14(1), 53-8.<br>* Whole abstract *<br>-- | 17-20 |
| X | CHEMICAL ABSTRACTS, vol. 90, no. 9, February 26, 1979<br>page 442, abstract 71236a<br>Columbus, Ohio, US<br>W.K. RYBAK et al. "Structure and catalytic reactivity of cobalt and molybdenum complexes in homogeneous oxidation reaction of hydrocarbons"<br>& PROC. CONF. COORD. CHEM. 1976, 6, 219-23<br>* Whole abstract * ./.<br>-- | 17-20 |

**CLASSIFICATION OF THE APPLICATION (Int. Cl.3)**

C 07 F 15/06
A 61 K 31/28
31/295
31/30
31/305
31/315//
C 07 C 53/126
53/124
53/122
53/10
53/06

**TECHNICAL FIELDS SEARCHED (Int. Cl.3)**

C 07 C 53/126
53/124
53/122
53/10
53/06
A 61 K 31/28
31/295
31/30
31/305
31/315

0049671

European Patent Office

# EUROPEAN SEARCH REPORT

Application number EP 81 40 1556

-3-

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| X | CHEMICAL ABSTRACTS, vol. 82, no. 10, March 10, 1975, page 712, abstract 67569d Columbus, Ohio, US J.J. ZIOLKOWSKI et al."Electronic structure and properties of the new oxotricobalt carboxylates" & BULL. ACAD. POL. SCI. SER. SCI. CHEM.1974, 22(10),895-900 * Whole abstract * -- | 17-20 | |
| X | CHEMICAL ABSTRACTS, vol. 77, no. 18, October 30, 1972 page 440, abstract 120504d Columbus, Ohio, US D. LUPU et al. "Moessbauer spectra and thermal electron transfer in mixed valence iron compounds" & J. INORG. NUCL. CHEM. 1972, 34 (9), 2803-10 * Whole abstract * -- | 17-20 | TECHNICAL FIELDS SEARCHED (Int. Cl.3) |
| X | JOURNAL OF THE CHEMICAL SOCIETY, Dalton Transactions, 1977 J. CATTERICK et al. "Synthesis magnetic properties, and Mössbauer spectra of polynuclear iron carboxylates", pages 1420-1425. * Pages 1422-1423 * -- | 17-20 | |
| | JOURNAL OF THE CHEMICAL SOCIETY, section A, 1966, no. 12 A. EARNSHAW et al. "Chemistry of polynuclear compounds. Part VI. Magnetic properties of trimeric chromium and iron carboxylates". pages 1656-1663 ./. | 17-20 | |

European Patent Office

# EUROPEAN SEARCH REPORT

Application number
EP 81 40 1556
-4-

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.3) |
|---|---|---|---|
| | * Page 1661 *<br>-- | | |
| X | JOURNAL OF THE CHEMICAL SOCIETY, section A, 1969<br>W.P. GRIFFITH "Tri- and tetra-nuclear oxy-complexes", pages 2270-2273<br>* Whole article *<br>-- | 17-20 | |
| X | DE - A - 2 239 615 (JOHNSON, MATTHEY & CO. LTD.)<br>* Claims 4,11; page 19, paragraph 3 *<br>-- | 17,18, 20 | TECHNICAL FIELDS SEARCHED (Int. Cl.3) |
| A | FR - M - 2 285 (YARDNEY INTERNATIONAL)<br>* Page 1, column 2, lines 1-25; claims *<br>-- | 1-4 | |
| D | BULLETIN OF THE CHEMICAL SOCIETY OF JAPAN, vol. 27, 1954, no. 2.<br>H. KOYAMA et al. "The crystal structure of zinc oxyacetate, $Zn_4O(CH_3COO)_6$", pages 112-114<br>* Whole article *<br>----- | 17-20 | |